# EUROPEAN PATENT APPLICATION

(11) **EP 3 811 878 A1**
(43) Date of publication of application: **28.04.2021**
(21) Application number: 18924080.7
(22) Date of filing: 25.06.2018
(51) Int. Cl.: A61B 17/128

(54) **VASCULAR CLIP**

(71) Applicant: Taiwan Surgical Corporation, Hsinchu County 302 (TW)
(72) Inventor: CHANG, Kuo-Yang, Taiwan 302 (TW)
(74) Representative: Cabinet Chaillot
(86) International application number: PCT/CN2018/092665
(87) International publication number: WO 2020/000140

(57) **Abstract**

A vascular clip includes a rail (231) in an outer tube (10) and having an elastic abutting front end, clip nails (30) in the rail (231), a clip-on nozzle (221) in front of the rail and passing out from the outer tube, a spring seat (41) at the rail, a guide bar (42) parallel to the rail and combined with the spring seat, a nail pushing device (43) slidably mounted on the rail and guided by the guide bar, a spring (44) sheathed on the guide bar for pushing the nail pushing device and clip nails, and a feeding push rod module (60) in the outer tube for pushing an upper nail push rod (50) of the frontmost clip nail and driving the clip-on nozzle to be clamped. When the upper nail push rod is operated to move forward/backward, the clip nail is sent into the clip-on nozzle and clamped. The vascular clip pushing the spring of the nail pushing device is guided by the guide bar, and thus will not be deformed to hinder the movement. Therefore, the nail feeding operation will be smooth.

## Description

### FIELD OF THE INVENTION

The present invention relates to the structure of a surgical instrument, and more particularly to a vascular clip with an effect of stable clip-pushing and smooth operation.

### BACKGROUND OF THE INVENTION

During laparoscopic surgery, blood vessels have to be closed in order to prevent blood loss from the incision at the surgical site, and surgical instruments for closing the blood vessels, such as vascular clip appliers are generally used for this purpose. During the use of the vascular clip applier, a long-rod shaped clipper applier is inserted into a human body from a cut opening in the surface of the human body, and a grip part is operated to drive the clip applier to clamp the vascular clips that are sequentially transported to the inner side of a clamping portion by using the clamping portion at the front end of the clip applier, and the vascular clips are clamped onto the blood vessels to close the blood vessels.

When the conventional vascular clip applier is used in a surgery, the clip applier at the front end is inserted into the human body as disclosed in Taiwan Pat. Application No. 104118985 (publication No. 201642812) entitled "Automatic clip applier", and the automatic clip applier comprises a clip applier installed in an outer sleeve, a clamping portion disposed at a front end of the clip applier, and a clamping unit operated with the clamping portion and provided for pushing each vascular clip forward by the force of a compressed spring, a first bushing and a second bushing sheathed on the front and rear positions of the rear end of the clip applier rear end respectively, wherein the forward movement of the first bushing is used to drive the clamping portion to clamp. In the front section of forward movement of the first bushing , the second bushing is driven to move rearward, and the second bushing will be released from the status of being synchronously moving with the first bushing in the rear section of the forward movement of the first bushing. At this time, the second bushing can be driven to move rearward, and drive the clamping unit is driven to push the vascular clip into an inner side of the clamping portion with the forward and rearward movements of the second bushing and the vascular clip is clamped by the clamping portion .

The clamping unit of the conventional automatic clip applier is propped and pushed by the compressed spring to achieve the effect of pushing multiple vascular clips forward. Since the spring for pushing the clamping unit is usually lack of a guiding design, the spring may be twisted easily in a relatively large compression condition, which will cause an unsmooth force of pushing the clamping unit and lead to an unsmooth movement of the vascular clip while the vascular clip applier is being operated. As a result, the vascular clip applier cannot be operated smoothly.

### SUMMARY OF THE INVENTION

In view of the aforementioned drawback of the conventional clip applier that has a spring without any guiding design which leads to an unsmooth motion of the spring while the clamping unit is pushed to clamp the vascular clip, the primary objective of the present invention is to provide a structure having a guide spring at the vascular clip applier to achieve the effect of operating the vascular clip applier more smoothly.

To achieve the aforementioned and other objectives, the present invention discloses a vascular clip comprising:
a nozzle rail having a nail channel formed in the outer tube, a rail formed in the nail channel, an elastic abutting end formed at a front end of the rail, a clip-on nozzle disposed in front of the nail channel, and protruding out from a front end of the outer tube, and the rail having a front end aligned precisely with a rear end of the clip-on nozzle and multiple clip nails arranged in a consecutive continuous manner in the rail, and a frontmost one of the clip nails abutting and positioned at the elastic abutting end;
a push element having a spring seat fixed to the rail at a position at a rear of the multiple clip nails, a guide bar combined with the spring seat and arranged parallelly to the rail, a nail pushing device slidably mounted on the rail, and a bushing formed on and protruding out from the nail pushing device and slidably sheathed on the guide bar, the nail pushing device having a front end pushing the rearmost clip nail, the guide bar having a compressed spring sheathed thereon, and both ends of the compressed spring abutting against the spring seat and the nail pushing device;
an upper nail push rod mounted in the outer tube and disposed on the top side of the nail channel, and having a nail toggling plate formed at a front end of the upper nail push rod, and a toggle end configured to be corresponsive to the frontmost clip nail and formed at a front end of the nail toggling plate; and
a feeding push rod module, having a feeding push rod installed in the outer tube, and a jaw clamp device combined with a front end of the feeding push rod, and provided for driving the clip-on nozzle to perform a clamping operation.

Further, the elastic abutting end has two elastic positioning plates formed on front and rear sides of the bottom of the nail channel respectively, and the two elastic positioning plates are plates upwardly warped from the front end, and the toggle end is an inverted U-shaped plate and staggered sideway with the two elastic positioning plates.

Further, the clip-on nozzle is in a tilted status, which is tilted downwardly to the front, an opening is formed in the front end of the rail, and an elastic guide plate configured to be corresponsive to the opening in position is formed at the middle of the bottom of the nail channel, and the elastic guide plate is a plate warped upwardly from the front end.

Further, the present invention further comprises two clip-on nozzle ribs respectively formed at left and right sides of a bottom surface of the clip-on nozzle, a head and a neck formed at front and rear positions of a front end of the feeding push rod respectively, a sliding groove concavely formed in a rear end surface of the jaw clamp device, and a necked portion formed at a rear end of the sliding groove, wherein the head has a front-to-rear length smaller than a front-to-rear length of the sliding groove, and the head is slidably installed in the sliding groove, and the neck is mounted through the necked portion, and has a front-to-rear extending length greater than a front-to-rear extending length of the necked portion, and a clamping groove is formed at the top of the jaw clamp device, is a tapered groove, and has a front opening and a front end sheathed around a rear end of the two clip-on nozzle ribs.

Further, the nozzle rail has a jaw pull rod fixed in the outer tube, is an elongated plate extending in both front and back directions, and has a pliers member disposed at a front end of the pull rod, the clip-on nozzle is bifurcated and formed at a front end of the pliers member, the nail channel is fixed an formed on the top of the jaw pull rod; a bushing opening configured to be corresponsive to the rail in position is formed at the middle of the upper nail pull rod and extending along a front-to-rear extending direction, for accommodating the guide bar and the bushing of the nail pushing device, and a driving element is combined with a rear end of the outer tube and is coupled to a rear a rear of the upper nail push rod and the feeding push rod module for driving the upper nail push rod and the feeding push rod module.

Preferably, the upper nail push rod and the feeding push rod have the rear ends backwardly protruding out from the rear end of the outer tube, an upper nail push rod rear-long-hole and an upper nail push rod hole are formed at front and rear positions of a rear end of the upper nail push rod, a feeding push rod hole is configured to be corresponsive to the upper nail push rod rear-long-hole in position and defiend through a rear end of the feeding push rod, a hook portion is formed at a rear end of the feeding push rod and extended backwardly, a notch is concavely formed in a position adjacent to a side of the hook portion, an inverted hook is formed at a rear end of the hook portion adjacent to the rear side of the notch and has a height smaller than a depth of the notch, and both front and back positions of the upper nail push rod hole are disposed within the range of the notch.

Preferably, the driving element at a rear end of the outer tube is combined with a slide bushing, he slide bushing is a tube extending in both front and rear directions, and the slide bushing has a front end sheathed around and fixed to a rear end of the outer tube, a rear end of the slide bushing mounted through a feeding push rod positioning pin long-hole is configured to be corresponsive to the upper nail push rod rear-long-hole and the feeding push rod hole in position, a feeding rod positioning pin long-hole mounted through the slide bushing is configured to be corresponsive to the upper nail push rod hole and the notch in position, a front end of the feeding rod positioning pin long-hole is provided with an inclined plane; and a feeding push rod slide bushing is sheathed around the outer tube at a position having the feeding push rod positioning pin long-hole, the feeding push rod slide bushing is mounted through a feeding push rod positioning pin hole, a feeding push rod positioning pin is inserted and fixed in the feeding push rod positioning pin hole, is mounted through the upper nail push rod rear-long-hole, and is inserted in the feeding push rod hole, a feeding rod slide bushing is sheathed around the slide bushing at a position having the feeding rod positioning pin long-hole, the feeding rod slide bushing is mounted through a feeding rod positioning pin hole, the feeding rod positioning pin hole is a long hole arranged in a direction same as the tangent direction of the slide bushing, and a feeding rod positioning pin is inserted in the feeding rod positioning pin hole, is mounted through the notch, and is inserted in the upper nail push rod hole.

Preferably, the slide bushing has a rear end mounted through a spring positioning pin hole, and a spring positioning pin is inserted and fixed in the spring positioning pin hole, the inverted hook is mounted and installed in a spring hook hole, a feeding spring is hooked between the spring hook hole and the spring positioning pin, and the feeding push rod positioning pin abuts against a rear end of the feeding push rod positioning pin long-hole.

In an application of the present invention, the rear end is coupled to a conventional or applicable driving element, and the driving element is used to drive the upper nail push rod to move forward, and the toggle end at the front end of the upper nail push rod is used to push the rail frontmost clip nail forward, so that the clip nail is separated from the elastic abutting end and enters into the clip-on nozzle of the front, and then the upper nail push rod is driven to move backward. At this time, the spring of the push element will push the nail pushing device to move forward and drive the clip nails to move forward until the frontmost clip nail is positioned to the elastic abutting end. After the frontmost clip nail enters into the clip-on nozzle, the driving element drives the feeding push rod to move forward and the feeding push rod is used to push the jaw clamp device to move forward and drive the clip-on nozzle to clamp the clip nail, so as to achieve the hemostatic effect of human tissues such as blood vessels by applying the clip nail.

The present invention has the following advantages:

The driving force of the push element for driving the clip nails to move forward along the rail comes from the spring. Since the spring is sheathed around the periphery of the guide bar, the spring can maintain applying forces to the nail pushing device in a straight line without any distortion that may hinder the movement, regardless of the level of the spring compression. In addition, the nail pushing device is guided by the guide bar to move forward linearly, so that the nail pushing device of the present invention can push the clip nails smoothly and stably. Thus, the clip nails can be pushed to move smoothly, and a smoother mechanical movement is provided, a better nail feeding operation is achieved when the blood vessel is clamped.

### BRIEF DESCRIPTION OF THE DRAWINGS

FIG. 1 is a perspective view of a preferred embodiment of the present invention;
FIG. 2 is an exploded perspective view of a preferred embodiment of the present invention;
FIG. 3 is a partially exploded perspective view of a preferred embodiment of the present invention;
FIG. 4 is an exploded perspective view of a driving element of a preferred embodiment of the present invention;
FIG. 5 is a top view of a preferred embodiment of the present invention;
FIG. 6 is a bottom view of a preferred embodiment of the present invention;
FIG. 7 shows operation views showing the movement at a rear section in accordance with a preferred embodiment of the present invention;
FIG. 8 shows operation views showing the movement of a clip nail pushed by an upper nail push rod in accordance with a preferred embodiment of the present invention;
FIG. 9 shows operational cross-sectional side views showing the movement of a clip nail pushed by an upper nail push rod in accordance with a preferred embodiment of the present invention; and
FIG. 10 shows operational views in partial section showing the movement of a clip-on nozzle driven to be clamped in accordance with a preferred embodiment of the present invention.

### BRIEF DESCRIPTION OF NUMERALS USED IN THE DRAWINGS

10: Outer tube; 11: Outer tube fixing long-hole; 20: Nozzle rail; 21: Jaw pull rod; 211: Jaw pull rod long-hole; 212: Jaw pull rod fixing hole; 22: Nozzle module; 221: Clip-on nozzle; 222: Clip-on nozzle rib; 23: Nail channel; 231: Rail; 232: Opening; 233: Elastic positioning plate; 234: Elastic guide plate; 30: Clip nail; 40: Push element; 41: Spring seat; 42: Guide bar; 43: Nail pushing device; 431: Bushing; 44: Spring; 50: Upper nail push rod; 51: Bushing opening; 52: Nail toggling plate; 521: Toggle end; 53: Upper nail push rod rear-long-hole; 54: Upper nail push rod hole; 55: Upper nail push rod front-long-hole; 60: Feeding push rod module; 61: Feeding push rod; 611: Feeding push rod hole; 612: Feeding push rod long-hole; 613: Hook portion; 614: Notch; 615: Inverted hook; 616: Spring hook hole; 617; Head; 618: Neck; 62: Jaw clamp device; 621: Sliding groove; 622: Necked portion ;623: Clamping groove; 70: Driving element; 71: Slide bushing; 711: Embedded block; 712: Fixed pin hole; 713: Feeding push rod positioning pin long-hole; 714: Feeding rod positioning pin long-hole; 715: Inclined plane; 716: Spring positioning pin hole; 72: Fixed pin; 73: Feeding push rod slide bushing; 731: Feeding push rod positioning pin hole; 74: Feeding push rod positioning pin; 75: feeding rod slide bushing; 751: Feeding rod positioning pin hole; 76: Feeding rod positioning pin; 77: Fixing cover; 771: Pin hole; 78: Spring positioning pin; 79: Feeding spring; A: Half-tube shell.

### DESCRIPTION OF THE PREFERRED EMBODIMENTS

To make it easier for our examiner and people having ordinary skill in the art to understand the objective of the invention, its structure, innovative features, and performance, we use preferred embodiments together with the attached drawings for the detailed description of the invention. It is intended that the embodiments and figures disclosed herein are to be considered illustrative rather than restrictive.

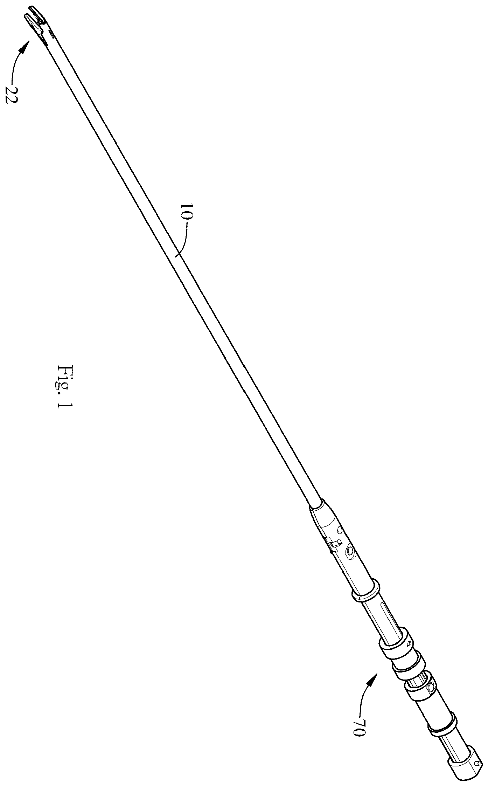

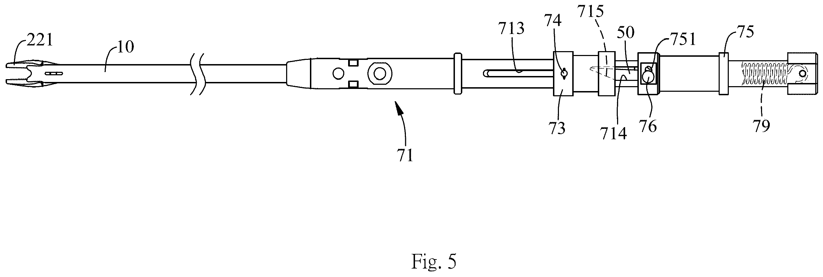

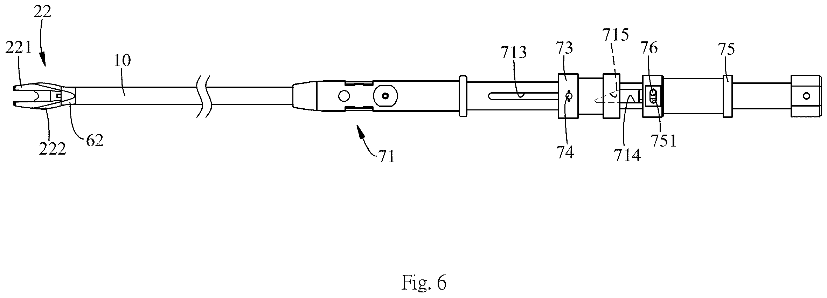

With reference to FIGS. 1 to 6, a preferred embodiment of a vascular clip applier in accordance with the present invention comprises an outer tube 10, a nozzle rail 20 mounted in the outer tube 10, multiple clip nails 30 disposed at the nozzle rail 20, a push element 40 disposed at the nozzle rail 20, an upper nail push rod 50 mounted in the outer tube 10, a feeding push rod module 60 mounted in the outer tube 10, and a driving element 70 coupled to a rear end of the outer tube 10, wherein:

The outer tube 10 is a round long tube extending in both front and rear directions, and an outer tube fixing long-hole 11 is radially defined through a rear end of the outer tube 10, and the outer tube fixing long-hole 11 is a long hole extending in both front and rear directions.

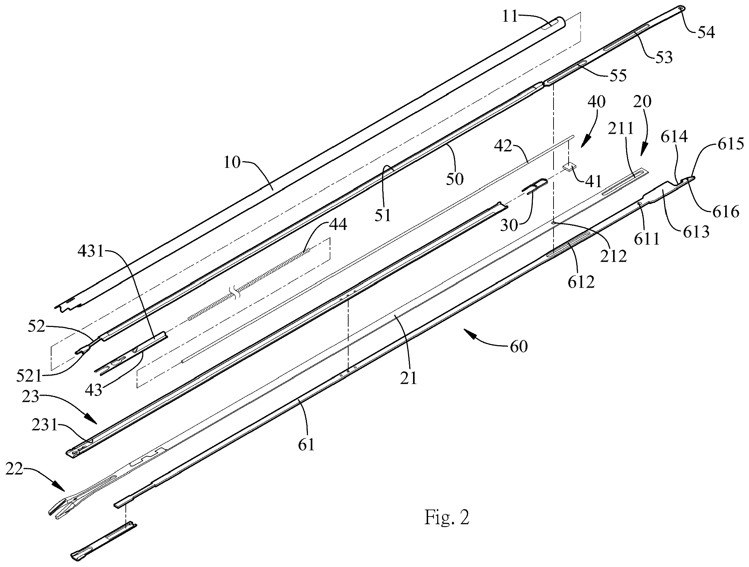

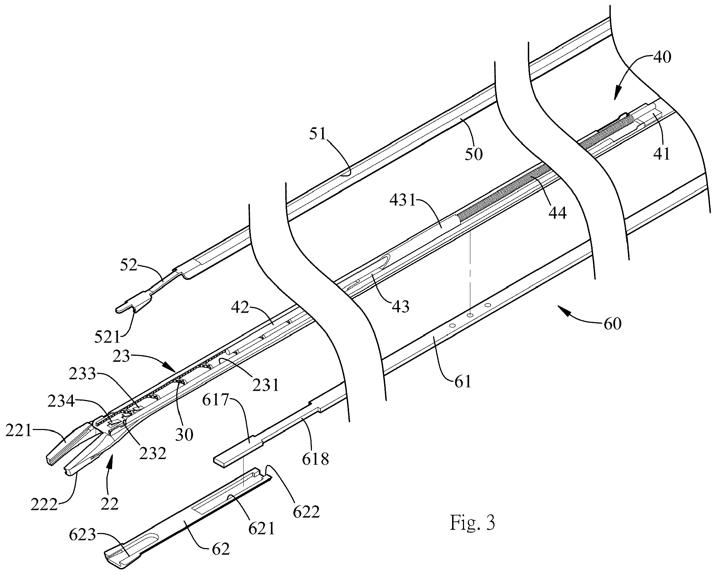

In FIGS. 2 and 3, the nozzle rail 20 comprises a jaw pull rod 21, a nozzle module 22, and a nail channel 23. The jaw pull rod 21 is an elongated plate extending in both front and rear directions and fixed in the outer tube 10, and the rear end of the jaw pull rod 21 protrudes rearwardly out from the rear end of the outer tube 10, and a jaw pull rod long-hole 211 is formed through the rear end of the jaw pull rod 21. In correspondence with the position of the outer tube fixing long-hole 11, a jaw pull rod fixing hole 212 is formed through the jaw pull rod 21. The pliers member 22 is coupled to a front end of the jaw pull rod 21, and the front end of the pliers member 22 is bifurcated to form a clip-on nozzle 221, and the clip-on nozzle 221 protrudes forwardly out from a front end of the outer tube 10, and the clip-on nozzle 221 is tilted downwardly at the front side. Two clip-on nozzle ribs 222 are respectively formed on the left and right sides of the bottom surface of the clip-on nozzle 221. The nail channel 23 is combined with and fixed to the top side of the jaw pull rod 21, and the nail channel 23 is a long channel extending in both front and rear directions. A rail 231 is formed in the nail channel 23 and extending in both front and rear directions. An opening 232 is formed at a front end of the rail 231.

The multiple clip nails 30 are arranged consecutively and continuously on the rail 231, and the front end of the rail 231 is aligned precisely with the rear end of the clip-on nozzle 221. In correspondence with the position of the front end of the rail 231, two elastic positioning plates 233 are formed on the front and rear sides of the middle of the bottom surface of the nail channel 23 respectively, and each elastic positioning plate 233 is a plate upwardly warped from the front end to form an elastic abutting end provided for passing each clip nail 30 unidirectionally forward by the two elastic positioning plates 233 to a position where the frontmost one of the clip nails 30 is abutted and positioned between the two elastic positioning plates 233. In correspondence with the position of the opening 232, an elastic guide plate 234 is formed at the middle of the bottom of the nail channel 23, wherein the elastic guide plate 234 is a plate upwardly warped from the front end and disposed in front of the two elastic positioning plates 233.

The push element 40 comprises a spring seat 41, a guide bar 42, a nail pushing device 43 and a spring 44. The spring seat 41 is combined with and fixed to a rear end of the rail 231 and is disposed at a rear end of the multiple clip nails 30. The guide bar 42 is a long rod extending in both front and rear directions, and has a rear end combined with the spring seat 41. The guide bar 42 is parallelly disposed on the top side of the rail 231. The nail pushing device 43 is slidably mounted on the rail 231, and has a bushing 431 is protruding out from the rail 231 and slidably sheathed on the guide bar 42., A front end of the nail pushing device 43 is provided for pushing the rearmost one of the clip nails 30. The spring 44 is in a compressed form and is mounted around the guide bar 42, and has two ends abut respectively against the spring seat 41 and the nail pushing device 43 to drive the nail pushing device 43 to push the clip nails 30.

The upper nail push rod 50 is an elongated sheet extending in both front and rear directions, is mounted in the outer tube 10 and is attached to the top side of the nail channel 23. In correspondence with the position of position of the rail 231, a bushing opening 51 is formed in the middle of the upper nail push rod 50. The bushing opening 51 is an elongated hole extending in both front and rear directions for accommodating the guide bar 42 and the bushing 431 of the nail pushing device 43. A nail toggling plate 52 is formed at a front end of the upper nail push rod 50, and a toggle end 521 is formed at a front end of the nail toggling plate 52. The toggle end 521 is an inverted U-shaped plate disposed on the rear side of the frontmost one of the clip nails 30 in the rail 231. The toggle end 521 and the two elastic positioning plates 233 are staggered sideway, so that when the toggle end 521 is moved forward or backward, the toggle end 521 will not produce an interference with the two elastic positioning plates 233 and the elastic guide plate 234.

The rear end of the upper nail push rod 50 protrudes rearwardly out from the rear end of the outer tube 10, and the front and rear positions of the rear end of the upper nail push rod 50 have an upper nail push rod rear-long-hole 53 and an upper nail push rod hole 54 respectively. The upper nail push rod rear-long-hole 53 is a long hole extending in both front and rear directions and superimposed with the jaw pull rod long-hole 211. In correspondence with the position of the outer tube fixing long-hole 11, an upper nail push rod front-long-hole 55 is defined through the upper nail push rod 50 and disposed at the front of the upper nail push rod rear-long-hole 53. The jaw pull rod fixing hole 212 is disposed under a front end of the upper nail push rod front-long-hole 55.

The feeding push rod module 60 comprises a feeding push rod 61 and a jaw clamp device 62. The feeding push rod 61 is an elongated sheet extending in both front and rear directions and is attached onto the bottom side of the jaw pull rod 21, and the rear end of the feeding push rod 61 protrudes out from the rear end of the outer tube 10. In correspondence with the position of the upper nail push rod rear-long-hole 53, a feeding push rod hole 611 is formed in a rear end of the feeding push rod 61. In correspondence with the position of the outer tube fixing long-hole 11, a feeding push rod long-hole 612 is formed through the feeding push rod 61. The jaw pull rod fixing hole 212 is disposed above the front end of the feeding push rod long-hole 612. A hook portion 613 extends rearwardly from the rear end of the feeding push rod 61. A notch 614 is concavely formed in a position near a side of the hook portion 613, wherein the front and rear positions of the upper nail push rod hole 54 are disposed within the range of the notch 614. The rear end of the upper nail push rod 50 is bent in a direction towards the inner side of the notch 614, so that the upper nail push rod hole 54 is disposed within the range of the inner side of the notch 614. An inverted hook 615 is formed at the rear end of the hook portion 613 and is coupled to a position adjacent to the rear side of the notch 614. The inverted hook 615 has a height smaller than the depth of the notch 614. A spring hook hole 616 is formed through the inverted hook 615. A head 617 with a larger sideway width and a neck 618 with a smaller sideway width are formed on the front and rear positions of the front end of the feeding push rod 61 respectively.

The jaw clamp device 62 is combined with a front end of the feeding push rod 61. The jaw clamp device 62 is an elongated block extending in both front and rear directions. A sliding groove 621 is concavely formed in the rear end of the jaw clamp device 62. A necked portion 622 having a front side with a sideway width smaller than that of other portions of the necked portion 622 is formed at a rear end of the sliding groove 621. The head 617 of the feeding push rod 61 is slidably installed in the sliding groove 621.,The front-to-rear length of the head 617 is smaller than the front-to-rear length of the sliding groove 621. The neck 618 is mounted through the necked portion 622., The front-to-rear length of the neck 618 is larger than the front-to-rear length of the necked portion 622. A clamping groove 623 is formed in the top of the jaw clamp device 62 and has a front opening. The clamping groove 623 is a groove tapered from the front to the rear. A front end of the clamping groove 623 is sheathed and combined with the rear end of the two clip-on nozzle ribs 222.

In FIGS. 2 and 4 to 6, the driving element 70 has a slide bushing 71, wherein the slide bushing 71 is a round tube extending in both front and rear directions and comprising upper and lower half-tube shells A engaged with each other. , Two embedded blocks 711 are formed on an inner peripheral surface in the front end of the slide bushing 71 and are diametrically opposite each other and each have a shape corresponding to the shape of the outer tube fixing long-hole 11.The embedded blocks 711 are embedded and positioned into the outer tube fixing long-hole 11, so that the front end of the slide bushing 71 is sheathed and fixed to the rear end of the outer tube 10. The slide bushing 71 has a fixed pin hole 712 defined through the slide bushing 71 at a position where is formed with the embedded blocks 711. A fixed pin 72 is mounted through the fixed pin hole 712, the front end of the upper nail push rod front-long-hole 55, and the front end of the feeding push rod long-hole 612. The middle of the fixed pin 72 is mounted through the jaw pull rod fixing hole 212 of the jaw pull rod 21 to fix the jaw pull rod 21 into the outer tube 10.

In correspondence with the positions of the upper nail push rod rear-long-hole 53, the jaw pull rod long-hole 211 and the feeding push rod hole 611, a feeding push rod positioning pin long-hole 713 is defined radially through the slide bushing 71. The feeding push rod positioning pin long-hole 713 is a long hole extending in both front and rear directions. In correspondence with the positions of the upper nail push rod hole 54 and the notch 614, a feeding rod positioning pin long-hole 714 is defined radially through the slide bushing 71. The feeding rod positioning pin long-hole 714 is a long hole extending in both front and rear directions. An inclined plane 715 is fromed on the front end of the feeding rod positioning pin long-hole 714.

A feeding push rod slide bushing 73 is installed around the slide bushing 71 at a position on where the feeding push rod positioning pin long-hole 713 is mounted. A feeding push rod positioning pin hole 731 is defined radially through the feeding push rod slide bushing 73. A feeding push rod positioning pin 74 is inserted and fixed into the feeding push rod positioning pin hole 731. The feeding push rod positioning pin 74 is mounted through the upper nail push rod rear-long-hole 53 and the jaw pull rod long-hole 211 and is inserted into the feeding push rod hole 611 of the feeding push rod 61, so that when the feeding push rod slide bushing 73 is moved forward, the feeding push rod positioning pin 74 can drive the feeding push rod 61 to move forward.

A feeding rod slide bushing 75 is sheathed on the slide bushing 71 at a position in which the feeding rod positioning pin long-hole 714 is defined. A feeding rod positioning pin hole 751 is defined radially through the feeding rod slide bushing 75, and the feeding rod positioning pin hole 751 is a long hole configured to be in a direction same as the tangent direction of the slide bushing 71. A feeding rod positioning pin 76 is inserted and fixed into the feeding rod positioning pin hole 751, is mounted through the notch 614, and is inserted into the upper nail push rod hole 54 at a rear end of the upper nail push rod 50.

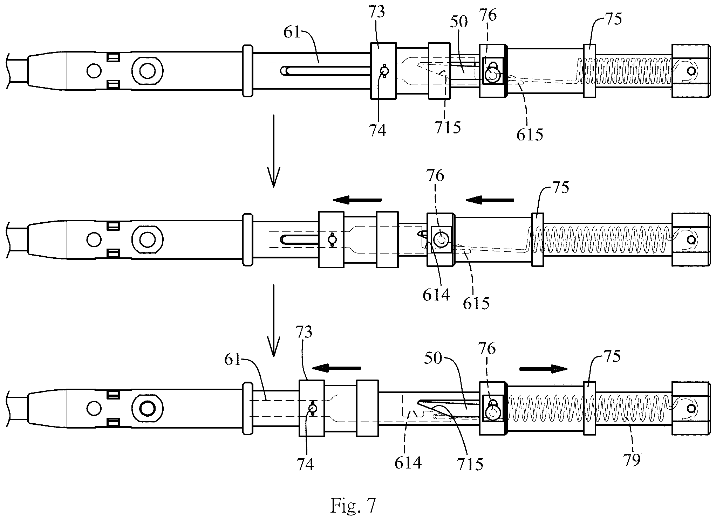

In FIG. 7, since the feeding rod positioning pin 76 abuts against an inner side of the notch 614, so that when the feeding push rod 61 is driven to move forward by the feeding push rod slide bushing 73, the inverted hook 615 on the rear end of the feeding push rod 61 can hook the feeding rod positioning pin 76. Thus, the upper nail push rod 50 and the feeding push rod 61 can move altogether. When the feeding rod positioning pin 76 is moved to the inclined plane 715, the feeding rod positioning pin 76 will be moved along the inclined plane 715 and will be separated from the inverted hook 615. At this time, the feeding rod slide bushing 75 and the feeding rod positioning pin 76 can be driven to move backward and to drive the upper nail push rod 50 to move backward. Accordingly, the upper nail push rod 50 is moved to the front and rear for one time in the aforementioned process. When the upper nail push rod 50 is moved to an original position and the feeding push rod 61 is also moved backward to an original position, the inverted hook 615 will be moved backward and pass through the feeding rod positioning pin 76. The feeding rod positioning pin 76 will fall into the notch 614 again. at this time, the inverted hook 615 is returned to a position where is capable of forwardly hooking the feeding rod positioning pin 76.

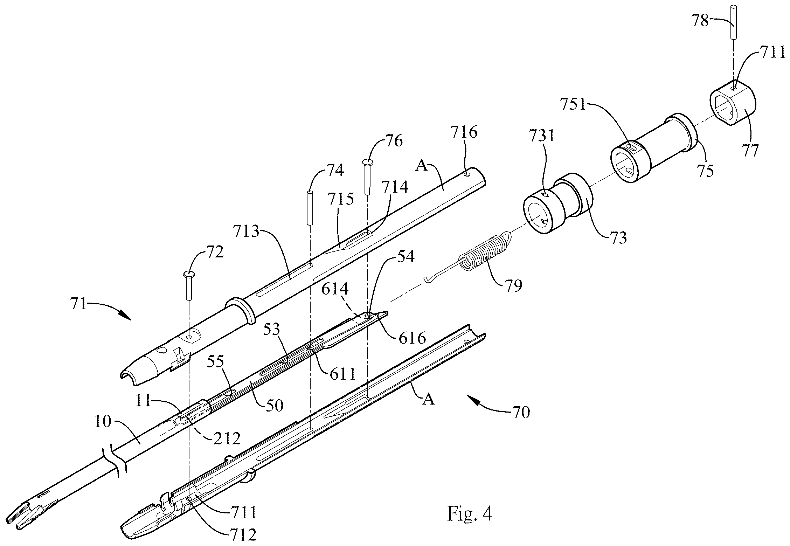

In FIG. 4, a spring positioning pin hole 716 is defined through the rear end of the slide bushing 71 , and the spring positioning pin hole 716 is defined through the rear ends of the two half-tube shells A. A fixing cover 77 is covered onto the rear ends of the two half-tube shells A, and a pin hole 771 is mounted radially through the fixing cover 77. A spring positioning pin 78 is mounted through the pin hole 771, and the spring positioning pin 78 is inserted into the spring positioning pin hole 716. A feeding spring 79 is hooked between the spring hook hole 616 in the rear end of the feeding push rod 61 and the spring positioning pin hole 716, so that the feeding push rod positioning pin 74 abuts against the rear end of the feeding push rod positioning pin long-hole 713.

In an application of the above preferred embodiment of the present invention, a holding unit of a conventional automatic clip applier is provided for driving the feeding push rod slide bushing 73 and the feeding rod slide bushing 75. However, the driving mechanism for driving the movement according to a preferred embodiment of the present invention is not limited to the conventional holding unit. It is noteworthy that any mechanism capable of driving the feeding push rod 61 and the upper nail push rod 50 to move in accordance with this preferred embodiment is the driving mechanism used in the present invention.

With reference to FIG. 7, in an application of the preferred embodiment of the present invention, the feeding push rod slide bushing 73 is driven to move forward and further to drive the feeding push rod 61 to move forward as well by the feeding push rod positioning pin 74. During the process of moving the feeding push rod 61 forward, the inverted hook 615 at the rear end of the feeding push rod 61 hooks the feeding rod positioning pin 76. Thus, the upper nail push rod 50 and the feeding push rod 61 can move forward altogether. When the feeding rod positioning pin 76 is moved forward to the inclined plane 715, the feeding rod positioning pin 76 will be moved along the inclined plane 715 and will be separated from the inverted hook 615. At this time, the feeding push rod 61 continues to move forward, and the driving mechanism drives the feeding rod slide bushing 75 to move backward. Consequently, the feeding rod slide bushing 75 can drive the upper nail push rod 50 to move backward by the feeding rod positioning pin 76. In other words. The upper nail push rod 50 performs one stroke (including the forward and backward movements) once during the whole process of moving the feeding push rod 61 forward.

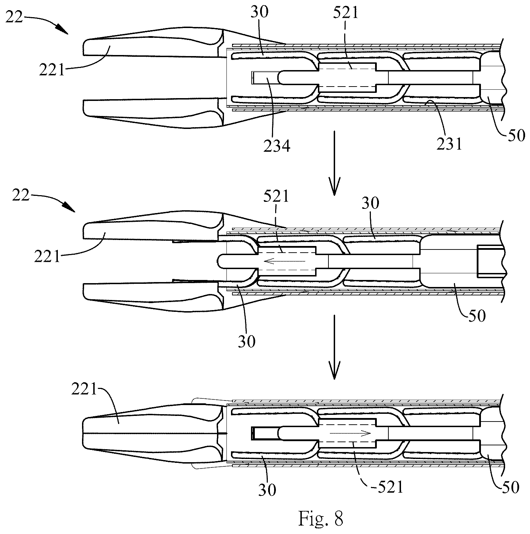

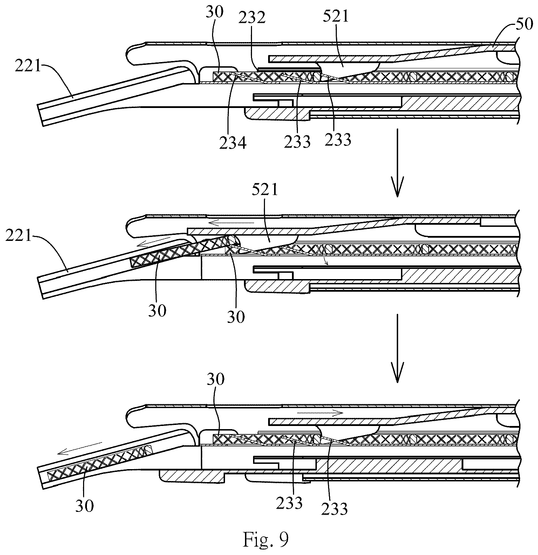

In FIGS. 8 and 9, in the stroke of moving the upper nail push rod 50 forward and backward for one time, the toggle end 521 at the front end of the upper nail push rod 50 will move the frontmost one of the clip nails 30 in the rail 231 forward, consequently, the frontmost clip nail 30 is pushed from the rear to the front into the clip-on nozzle 221, and then the upper nail push rod 50 is moved backward to let the clip nail 30 be remained in the clip-on nozzle 221 and be clamped by the clip-on nozzle 221 to achieve the effect of clamping the blood vessel. When the frontmost clip nail 30 is pushed by the toggle end 521 of the upper nail push rod 50 as shown in FIG. 3, the push element 40 keeps applying forces provided by the spring 44 to push the nail pushing device 43 to move forward along the rail 231. When the frontmost clip nail 30 is moved forward and is separated from the position between the two elastic positioning plates 233, the second one of the clip nails 30 and the others of clip nails 30 will be pushed by the nail pushing device 43 to move forward until the second clip nail 30 is moved between the two elastic positioning plates 233 and temporarily fixed to become the frontmost clip nail 30 in the rail 231.

During the process for the push element 40 pushing the nail pushing device 43 forward by the spring 44, the spring 44 guided by the guide bar 42 can be pushed forward only., Because the spring 44 is mounted around the periphery of the guide bar 42, the spring 44 will not be twisted or deformed into an S-shape to hinder the movement regardless of the extent of compression of the compressed spring 44. Accordingly, the spring 44 can push the nail pushing device 43 forward to move the clip nails 30 forward smoothly, and the present invention can provide a smoother mechanical movement. In the present invention, the elastic guide plate 234 is installed on the opening 232 at the front end of the rail 231. With reference to FIG. 9, during the process of pushing the frontmost clip nail 30 forward into the clip-on nozzle 221, and the rear end of the multiple clip nails 30 is pushed by the elastic guide plate 234 to warp upward, thus, the clip nails 30 can be tilted to fit with the angle of inclination of the clip-on nozzle 221, and the clip nails 30 can be moved into the clip-on nozzle 221 smoothly to avoid the clip nails 30 from getting stuck in the opening 232.

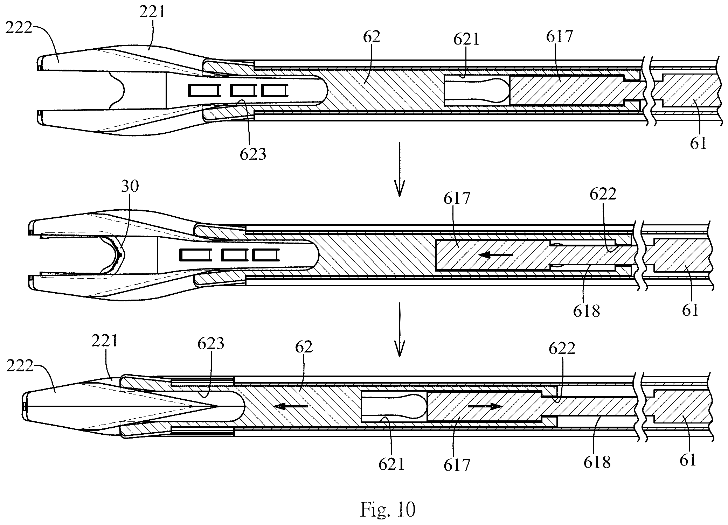

During the process of moving the feeding push rod 61 forward as shown in FIG. 10, when the feeding push rod 61 starts to move forward, the head 617 at a front end of the feeding push rod 61 slides forward in the sliding groove 621 of the jaw clamp device 62. At this time, the feeding push rod 61 will not drive the jaw clamp device 62 to move forward. After the frontmost clip nail 30 is sent into the clip-on nozzle 221, the head 617 of the feeding push rod 61 is pushed onto a front end surface of the sliding groove 621. At this time, the feeding push rod 61 drives the jaw clamp device 62 to move forward, and uses the two clip-on nozzle ribs 222 are force to be moved closely to each other by the clamping groove 623 of the jaw clamp device 62. Consequently, the clip nail 30 can be clamped in the clip-on nozzle 221. When the feeding push rod 61 is moved backward, the head 617 slides backward in the sliding groove 621 of the jaw clamp device 62. At this itme, the feeding push rod 61 will not backwardly drive the jaw clamp device 62 to move backward and can maintain the clamped status of the clip-on nozzle 221 for a period of time. After the feeding push rod 61 is moved backward again, the head 617 backwardly hooks the necked portion 622 of the jaw clamp device 62 to drive the jaw clamp device 62 to move backward. Consequently, the clip-on nozzle rib 222 is released, and the clip-on nozzle 221 is situated in an open status.

While the invention has been described by means of specific embodiments, numerous modifications and variations could be made thereto by those skilled in the art without departing from the scope and spirit of the invention as set forth in the claims.

## Claims

1. A vascular clip, **characterized in that**:
a nozzle rail (20) has a nail channel (23) formed in an outer tube (10), a rail (231) formed in the nail channel (23), an elastic abutting end formed at a front end of the rail (231), and a clip-on nozzle (221) disposed in front of the nail channel (23) and protruding out from a front end of the outer tube (10), and the rail (231) has a front end aligned precisely with a rear end of the clip-on nozzle (221) and multiple clip nails (30) arranged in a consecutive continuous manner in the rail (231), and a frontmost one of the clip nails (30) is abutted and positioned at the elastic abutting end;
a push element (40) has a spring seat (41) fixed to the rail (231) at a position at a rear end of the multiple clip nails (30), a guide bar (42) combined with the spring seat (41) and arranged parallelly to the rail (231), a nail pushing device (43) slidably mounted on the rail (231), and a bushing (431) formed on and protruding out from the nail pushing device (43) and slidably sheathed on the guide bar (42), the nail pushing device (43) has a front end that pushes the rearmost clip nail (30), the guide bar (42) has a compressed spring (44) sheathed thereon, and both ends of the compressed spring (44) abut against the spring seat (41) and the nail pushing device (43) respectively;
an upper nail push rod (50) is mounted in the outer tube (10) and disposed on the top side of the nail channel (23) and has a nail toggling plate (52) formed at a front end of the upper nail push rod (50), and a toggle end (521) configured to be corresponsive to the frontmost clip nail (30) and formed at a front end of the nail toggling plate (52); and
a feeding push rod module (60) has a feeding push rod (61) installed in the outer tube (10), and a jaw clamp device (62) combined with a front end of the feeding push rod (61) and provided for driving the clip-on nozzle (221) to perform a clamping operation.

2. The vascular clip as claimed in claim 1, wherein the elastic abutting end has two elastic positioning plates formed on front and rear sides of the bottom of the nail channel (23) respectively, and the two elastic positioning plates are plates upwardly warped from the front end, and the toggle end (521) is an inverted U-shaped plate and staggered sideway with the two elastic positioning plates (233).

3. The vascular clip as claimed in claim 1, wherein the clip-on nozzle (221) is in a tilted status, which is tilted downwardly to the front, an opening (233) is formed in the front end of the rail (231), an elastic guide plate (234) configured to be corresponsive to the opening (233) in position is formed at the middle of the bottom of the nail channel (23), and the elastic guide plate (234) is a plate warped upwardly from the front end.

4. The vascular clip as claimed in claim 3, further comprising two clip-on nozzle ribs (222) are respectively formed on left and right sides of a bottom surface of the clip-on nozzle (221), a head (617) and a neck (618) formed at front and rear positions of a front end of the feeding push rod (61) respectively, a sliding groove (621) concavely formed in a rear end surface of the jaw clamp device (62), and a necked portion (622) formed at a rear end of the sliding groove (621), wherein the head (617) has a front-to-rear length smaller than a front-to-rear length of the sliding groove (621), the head (617) is slidably installed in the sliding groove (621), the neck (618) is mounted through the necked portion (622), and has a front-to-rear extending length greater than a front-to-rear extending length of the necked portion (622), and a clamping groove (623) is formed in the top of the jaw clamp device (62), is a tapered groove, and has a front opening and a front end sheathed around a rear end of the two clip-on nozzle rib (222) .

5. The vascular clip as claimed in claim 4, wherein the nozzle rail (20) has a jaw pull rod (21) fixed in the outer tube (10), is an elongated plate extending in both front and rear directions, and has a pliers member (22) disposed at a front end of the pull rod (21), the clip-on nozzle (221) is bifurcated and formed at a front end of the pliers member (22), the nail channel (23) is fixed an formed on the top of the jaw pull rod (21), a bushing opening (51) configured to be corresponsive to the rail (231) in position is formed at the middle of the upper nail pull rod and extending along a front-to-rear extending direction for accommodating the guide bar (42) and the bushing (431) of the nail pushing device (43), and a driving element (70) is combined with a rear end of the outer tube (10) and is coupled to a rear of the upper nail push rod (50) and the feeding push rod module (60) for driving the upper nail push rod (50) and the feeding push rod module (60).

6. The vascular clip as claimed in claim 5, wherein the upper nail push rod (50) and the feeding push rod (61) have the rear ends backwardly protruding out from the rear end of the outer tube (10), an upper nail push rod rear-long-hole (53) and an upper nail push rod hole (54) are formed at front and rear positions of a rear end of the upper nail push rod (50), a feeding push rod hole (611) is configured to be corresponsive to the upper nail push rod rear-long-hole (53) in position and defined through a rear end of the feeding push rod (61), a hook portion (613) is formed at a rear end of the feeding push rod (61) and extending backwardly, a notch (614) is concavely formed in a position adjacent to a side of the hook portion (613), an inverted hook (615) is formed at a rear end of the hook portion (613) adjacent to the rear side of the notch (614) and has a height smaller than a depth of the notch (614), and both front and back positions of the upper nail push rod hole (54) are disposed within the range of the notch (614).

7. The vascular clip as claimed in claim 6, wherein the driving element (70) at a rear end of the outer tube (10) is combined with a slide bushing (71), the slide bushing (71) is a tube extending in both front and rear directions and has a front end sheathed around and fixed to a rear end of the outer tube (10), a rear end of the slide bushing (71) mounted through a feeding push rod positioning pin long-hole (713) is configured to be corresponsive to the upper nail push rod rear-long-hole (53) and the feeding push rod hole (611) in position, a feeding rod positioning pin long-hole (714) (714) mounted through the slide bushing (71) is configured to be corresponsive to the upper nail push rod hole (54) and the notch (614) in position, a front end of the feeding rod positioning pin long-hole (714) is provided with an inclined plane (715), a feeding push rod slide bushing (73) is sheathed around the outer tube (10) at a position having the feeding push rod positioning pin long-hole (713), the feeding push rod slide bushing (73) is mounted through a feeding push rod positioning pin hole (731), and a feeding push rod positioning pin (74) is inserted and fixed in the feeding push rod positioning pin hole (731), is mounted through the upper nail push rod rear-long-hole (53), and is inserted in the feeding push rod hole (611), a feeding rod slide bushing (75) is sheathed around the slide bushing (71) at a position having the feeding rod positioning pin long-hole (714) and is mounted through a feeding rod positioning pin hole (751), the feeding rod positioning pin hole (751) is a long hole arranged in a direction same as the tangent direction of the slide bushing (71), and a feeding rod positioning pin (76) is inserted in the feeding rod positioning pin hole (751), is mounted through the notch (614), and is inserted in the upper nail push rod hole (54).

8. The vascular clip as claimed in claim 7, wherein the slide bushing (71) has a rear end mounted through a spring positioning pin hole (716), and a spring positioning pin (78) is inserted and fixed in the spring positioning pin hole (716), the inverted hook (615) is mounted and installed in a spring hook hole (616), a feeding spring (79) is hooked between the spring hook hole (616) and the spring positioning pin (78), and the feeding push rod positioning pin (74) abuts against a rear end of the feeding push rod positioning pin long-hole (713).
